# EUROPÄISCHE PATENTSCHRIFT

(11) **EP 2 187 781 B1**
(45) Veröffentlichungstag und Bekanntmachung des Hinweises auf die Patenterteilung: **23.03.2011**
(21) Anmeldenummer: 08803678.5
(22) Anmeldetag: 04.09.2008
(51) Int. Cl.: A47C 7/14, A61B 19/00, A61G 15/08

(54) **STUHL, INSBESONDERE EIN ZAHNARZTSTUHL, AUFWEISEND EINE SITZFLÄCHE MIT NEIGBAREN BEINAUFLAGEN**
CHAIR, PARTICULARLY A DENTIST CHAIR, HAVING A SEAT COMPRISING INCLINABLE LEG RESTS
FAUTEUIL, NOTAMMENT FAUTEUIL DE DENTISTE, PRÉSENTANT UNE ASSISE AVEC DES APPUIE-JAMBES INCLINABLES

(30) Priorität: 05.09.2007 DE 102007042032
(43) Veröffentlichungstag der Anmeldung: 26.05.2010
(73) Patentinhaber: Sirona Dental Systems GmbH, 64625 Bensheim (DE)
(72) Erfinder: MEILLER, Hermann, 92533 Wernberg/Köblitz (DE); JAHN, Wolfram, 92286 Vilshofen (DE)
(74) Vertreter: Sommer, Peter
(86) Internationale Anmeldenummer: PCT/EP2008/061708
(87) Internationale Veröffentlichungsnummer: WO 2009/030732

(56) Entgegenhaltungen:
- WO-A-95/34234
- CA-A1- 2 567 549
- DE-A1- 2 905 065

## Beschreibung

### Technisches Gebiet

Die Erfindung betrifft einen Stuhl, insbesondere ein Zahnarztstuhl, der eine im Wesentlichen horizontal ausgerichtete Sitzfläche mit einer Sitzplatte zur Aufnahme des Gesäßes einer Person aufweist. Weiterhin sind mindestens zwei, sich an die Sitzplatte anschließende Beinauflagen vorhanden, die gegenüber der Sitzplatte um einen Winkel α neigbar sind.

Eine Neigung der Beinauflage und somit des Oberschenkels ermöglicht es dem Zahnarzt während einer Behandlung am Patientenstuhl, wenn die Rückenlehne des Patientenstuhls sehr weit nach hinten geneigt ist, zumindest einen Oberschenkel unter der Rückenlehne des Patientenstuhls zu positionieren, um die erforderliche Nähe zum Patienten zu erreichen. Ohne den Oberschenkel gegenüber der Horizontalen zu neigen, würde der Zahnarzt sein Bein nicht unter der Rückenlehne positionieren und nicht die erforderliche Position erreichen können.

### Stand der Technik

Aus der WO 01/00061 A1 ist ein Stuhl bekannt, der zwei separate Schenkelflügel aufweist. Diese beiden Flügel stellen separate Stützen für die Schenkel eines Benutzers dar, wobei sie individuell federbelastet sein können, was, wie es dort ausdrücklich heißt, aber nicht dargestellt ist.

Aus der WO 9610937 ist ein Sitz mit sattelförmiger Sitzfläche bekannt, die sich aus einem hinteren Teil und aus zwei vorderen Schenkelflügeln und einem zwischen diesen Flügeln dazwischen angeordneten feststehenden Mittelteil zusammensetzt. Die Schenkelflügel sind aus einer Neutralposition, in der alle Teile der Sitzfläche in derselben Ebene liegen, schwenkbar in eine Benutzungsposition, in der die beiden Flügel und der hintere Teil der Sitzfläche gegenüber dem feststehenden Mittelteil je nach dem betreffenden Bedürfnis des Benutzers verschwenkt sind. Die Teile der Sitzfläche sind wieder elastisch in die Null-Stellung rückstellbar, wofür eine elastische Gegenkraft vorgesehen ist, gegen die die schwenkbaren Teile aus der Null-Stellung um die Achsen schwenkbar sind, um die Sitzflächen einer Änderung der Sitzposition des Benutzers, bei der der Benutzer die Winkelstellung seiner Hüften ändert, anzupassen.

Aus der DE 1 779 707 A ist ein Kraftfahrzeugsitz mit einem linken und einem rechten schwenkbaren Beinstützteil bekannt, die beide unabhängig von einander einstellbar und jeweils über einen separaten Antriebsmechanismus schwenkbar sind. Jedes Beinstützteil hat einen separaten Einstell- und Federmechanismus. Der Mechanismus ist darauf abgestellt, dass beim Schwenken des jeweiligen Beinstützteils seine Frontkante sich nicht vor- und zurückverschiebt.

Aus der DE 800 488 C ist ein Versehrtenstuhl mit verstellbarer Stützsäule zur Höheneinstellung bekannt. Am vorderen Rand der Sitzfläche sind zwei separate Beinschalen schwenkbar gelagert angeordnet, wobei diese Beinschalen gegeneinander austauschbar und federnd und gelenkartig einstellbar sein können. Die Beinschalen dienen zur Auflage von Prothesen.

Aus der DE 693 06 938 T2 ist ein Sitz mit einer Sitzfläche und daran gelagerten separaten schwenkbaren Klappen bekannt, von denen jede mit einem separaten Antrieb verbunden und über eine separate Steuereinrichtung separat steuerbar ist. Es ist hierbei vorgesehen, dass der Antrieb von Hand durch Drehen an einem Steuerrad erfolgt. Bei anderen abgewandelten Ausführungen kann die Antriebsvorrichtung auch einen pneumatischen Kolben oder einen Elektromotor umfassen.

Aus der CA 2 567 549 A1 ist ein Stuhl mit einer horizontalen Sitzfläche und mit zwei mit der Sitzfläche verbundenen beweglichen Beinauflagen bekannt. Die beiden Beinauflagen, sind auf einer Wippe abgestützt, wobei die Wippe beim Verschwenken einer der beiden Beinauflagen nach unten eine Anhebung der anderen Beinauflage relativ zu der nach unten verschwenkten Beinauflage nach oben bewirkt.

Aufgabe ist es, einen Stuhl derart weiterzubilden, dass eine ergonomisch besser angepasste und individuelle Neigung der Beinauflage möglich ist und gleichzeitig eine stabile Sitzhaltung einer Person, insbesondere eines Zahnarztes erreicht wird.

### Darstellung der Erfindung

Erfindungsgemäß weist der Stuhl, insbesondere ein Zahnarztstuhl, eine im Wesentlichen horizontal ausgerichtete Sitzfläche mit einer Sitzplatte zur Aufnahme des Gesäßes.einer Person auf. Weiterhin umfasst der Stuhl mindestens zwei sich an die Sitzplatte anschließende Beinauflagen, die gegenüber der Sitzplatte um einen Winkel neigbar sind. Die beiden Beinauflagen stützen sich auf einer unterhalb derselben angeordneten und mit der Sitzplatte zumindest mittelbar verbundenen Wippe ab, wobei die Wippe beim Verschwenken einer der beiden Beinauflagen nach unten eine Anhebung der anderen Beinauflage relativ zu der nach unten verschwenkten Beinauflage nach oben bewirkt. Beide Beinauflagen sind gegenüber der Sitzplatte um gleichzeitig einen Winkel α und eine der beiden Beinauflagen um einen an den Winkel α anschließenden Winkel β neigbar. Die Wippe bewirkt beim Verschwenken einer der beiden Beinauflagen über den Winkel α hinaus nach unten eine Anhebung der anderen Beinauflage, relativ zu der über den Winkel α hinaus nach unten verschwenkten Beinauflage nach oben.

Dadurch wird erreicht, dass die Position der Oberschenkel rechts oder links ergonomisch und individuell einstellbar ist und durch das proportionale Anheben der einen Beinauflage der Arzt nicht nach vorne von der Sitzplatte abrutscht. Da ein gewisses Maß der Neigung einer Beinauflage von der Neigung der jeweils anderen Beinauflage abhängig ist, bleibt dem Arzt die sich aufrichtende Beinauflage zum abstützen, auch wenn der andere Oberschenkel weit nach unten geneigt, unter der Rückenlehne des Patientenstuhls positioniert ist.

Zunächst können beide Beinauflagen um gleichzeitig ein gewisses Maß, nämlich den Winkel α, nach unten geneigt werden, bevor der proportionale Ausgleich über die Wippe erfolgt. Hierbei ist in vorteilhafter Weise vorgesehen, die Beinauflage, die angehoben wird, nicht über die Sitzplatte hinaus anzuheben. Dies wird dadurch erreicht, dass die Anschläge so eingestellt werden, dass der Winkel β ungefähr so groß ist wie der Winkel α.

Vorteilhafterweise kann die Wippe über ein Drehlager an einem Hauptträger der Sitzplatte um eine Schwenkachse schwenkbar gelagert sein.

Vorteilhafterweise können die beiden Beinauflagen jeweils gegen eine Federkraft eines Federelements gegenüber der Sitzplatte verschwenkbar sein. Dabei kann die Wippe gegen eine Federkraft zumindest eines Federelements gegenüber der Sitzplatte verschwenkbar sein, wobei die zur Verstellung der Beinauflagen erforderliche Kraft kleiner als die zur Verstellung der Wippe erforderliche Kraft ist. Vorteilhafterweise kann die Wippe Auflagearme für mindestens jeweils eine Beinauflage aufweisen, wobei die Auflagearme in ihrer Höhe gegenüber der Wippe verstellbar sein können, insbesondere gegen eine Federkraft.

Vorteilhafterweise kann die Wippe zwei Bolzen aufweisen, über die die Wippe an dem jeweiligen Federelement angelenkt ist, wobei das Federelement in einer Federkammer des Hauptträgers angeordnet sein kann und der Bolzen über eine Federkammeröffnung in einer Richtung um die Schwenkachse in die Federkammer eingeführt werden kann.

Vorteilhafterweise kann der Auflagearm über einen Bolzen um eine Achse schwenkbar an der Wippe gelagert sein.

Vorteilhafterweise können die beiden Beinauflagen jeweils gegen eine Federkraft eines Federelements gegenüber der Sitzplatte verschwenkbar sein. Dabei kann die Wippe zumindest gegen eine Federkraft zumindest eines Federelements gegenüber der Sitzplatte höhenverstellbar sein, wobei die zur Verstellung der Beinauflagen erforderliche Kraft kleiner als die zur Verstellung der Wippe erforderliche Kraft sein kann.

Vorteilhafterweise kann die Wippe beidseitig der Schwenkachse auf jeweils einem Mitnehmer aufgelegt werden, wobei die Mitnehmer um die Schwenkachse oder parallel zu der Schwenkachse schwenkbar gelagert sein können. Zudem sind die Mitnehmer in Richtung um ihre Schwenkachse über die Federelemente gegenüber der Sitzplatte oder dem Hauptträger vorgespannt und können in Richtung der Schwenkachse gegen einen am Hauptträger befestigten Bolzen angeschlagen werden.

Vorteilhafterweise kann die Wippe höhenverstellbar sein und kann für die Höhenverstellung über das Drehlager in einer vertikal verlaufenden Längsnut am Hauptträger gelagert sein. Die Wippe oder das Drehlager kann dabei über das Federelement in Richtung der Längsnut vorgespannt sein, wodurch ein gleichzeitiges Absenken der beiden Beinauflagen ermöglicht wird.

Vorteilhafterweise kann die Verstellung der Beinauflagen durch einen oder mehrere Anschläge begrenzt sein. Auch kann die Verstellung der Wippe durch einen oder mehrere Anschläge begrenzt sein, wobei der oder die Anschläge zur Verstellung der Beinauflagen von dem oder den Anschlägen zur Verstellung der Wippe verschieden sind.

Vorteilhafterweise kann die jeweilige Beinauflage über ein Scharnier jeweils um eine Schwenkachse an der Sitzplatte schwenkbar gelagert sein. Dabei können die beiden Schwenkachsen koaxial oder in einem Winkel δ zwischen 135 und 179 Grad, insbesondere zwischen 160 und 164 Grad zueinander angeordnet sein. Durch die winklige Anordnung kann eine noch bessere ergonomische Anpassung der Oberschenkel erreicht werden. Insbesondere lässt sich eine noch bessere Positionierung des Oberschenkels unter der Rückenlehne des Patientenstuhls erreichen.

Hierzu ist vorteilhaft, wenn das Maß des Winkels α und des Winkels β und/oder die Federkräfte manuell einstellbar sind.

Verstellen der Neigungswinkel von den beiden Beinauflagen eines Stuhls, wie er vorstehend beschrieben ist. Ausgehend von einer Grundposition "0", in der beide Beinauflagen den geringsten Neigungswinkel gegenüber der Sitzplatte aufweisen, weist das Verfahren folgende Verfahrensschritte auf:
Durch die Belastung einer oder beider Beinauflagen werden beide Beinauflagen in Bezug zu der Sitzplatte entgegen einer Federkraft gleichzeitig um einen Winkel α von maximal 9 Grad in eine Zwischenposition "1" geneigt. Dadurch kann sich der Arzt mit dem gesamten Oberkörper weiter nach vorne, Richtung Patient lehnen.

Durch eine weitere Belastung einer der beiden Beinauflagen und einer daraus resultierenden Bewegung der Beinauflage entgegen einer Federkraft über den Winkel α hinaus nach unten wird die andere Beinauflage relativ zu der über den Winkel α hinaus nach unten geneigten Beinauflage nach oben bewegt. Der Arzt neigt eine Beinauflage, um mit einem Oberschenkel unter die Rückenlehne zu kommen und stützt sich mit dem anderen Oberschenkel auf der sich nach oben bewegenden Beinauflage ab.

Die eine Beinauflage wird so lange nach unten bewegt, bis die andere nach oben bewegte Beinauflage den gleichen Neigungswinkel gegenüber der Sitzplatte aufweist wie in der Grundposition "0", bis eine Endposition "2" erreicht ist. Dadurch wird eine Überstreckung des Oberschenkels nach oben hin vermieden.

Wenn die Beinauflagen die Endposition "2" erreicht haben, wird durch mehrere Anschläge vermieden, dass sich die eine Beinauflage weiter anhebt und dass sich die andere Beinauflage weiter absenkt. Diese Endposition "2" ist stabil, der Arzt braucht die beiden Beinauflagen nicht selbst stabilisieren.

### Kurze Beschreibung der Zeichnungen

Der erfindungsgemäße Stuhl wird anhand der Zeichnung erläutert. Es zeigt:
- Fig. 1: eine mehrteilige Sitzfläche eines Zahnarztstuhls,
- Fig. 2: eine Ansicht der Sitzfläche aus Fig. 2 von unten mit einem Hauptträger und einer Trägeranordnung,
- Fig. 3: die Trägeranordnung aus Fig. 2 mit einer Wippe,
- Fig. 4A: eine Grundstellung der Wippe in Bezug auf den Hauptträger,
- Fig. 4B: eine ausgelenkte Stellung der Wippe in Bezug auf den Hauptträger,
- Fig. 5: einen Schnitt entlang der Linie AA aus Fig. 3,
- Fig. 6: eine Seitenansicht der am Hauptträger angeordneten Sitzplatte mit einer Abstützung der Beinauflagen,
- Fig. 7A: ein weiteres Ausführungsbeispiel der Erfindung in einer Seitenansicht,
- Fig. 7B: das weitere Ausführungsbeispiel aus Fig. 7A in ei- ner Ansicht von vorn,
- Fig. 8A: eine erste Zwischenposition "1" in einer Seitenan- sicht
- Fig. 8B: die erste Zwischenposition "1" in einer Ansicht von vorn,
- Fig. 9A: eine Endposition "2" in einer Seitenansicht
- Fig. 9B: die Endposition "2" in einer Ansicht von vorn,
- Fig.: die in diesem Ausführungsbeispiel über Federele- mente an dem Hauptträger aufgehängte Wippe im De- tail,
- Fig. 11: das Ausführungsbeispiel aus Fig. 2 im Detail;
- Fig. 12: eine Ansicht einer Sitzfläche von oben mit abge- winkelten Schwenkachsen für die Beinauflagen.

### Ausführungsbeispiele

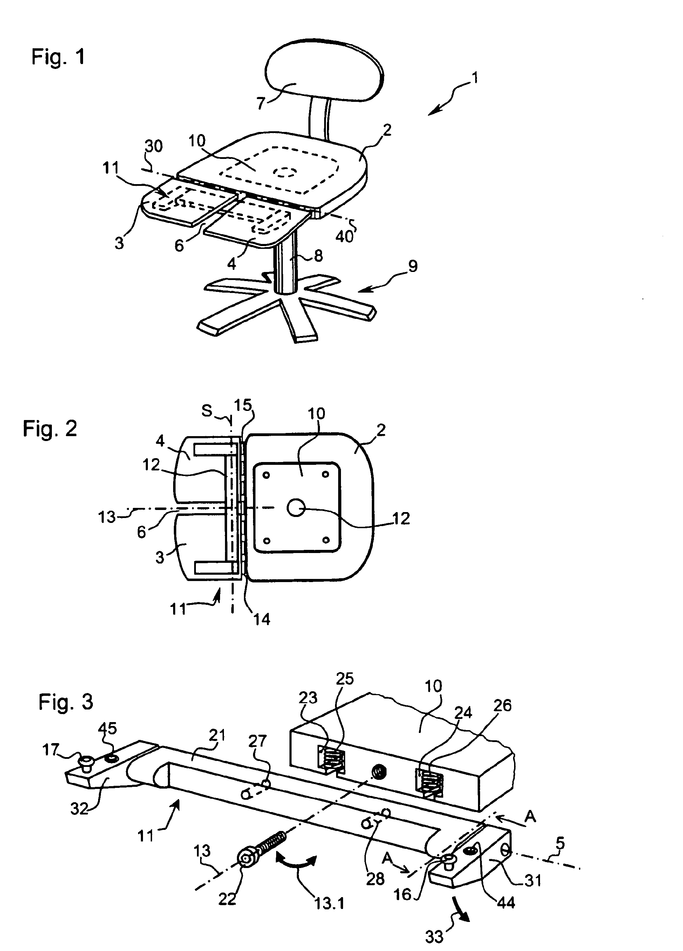

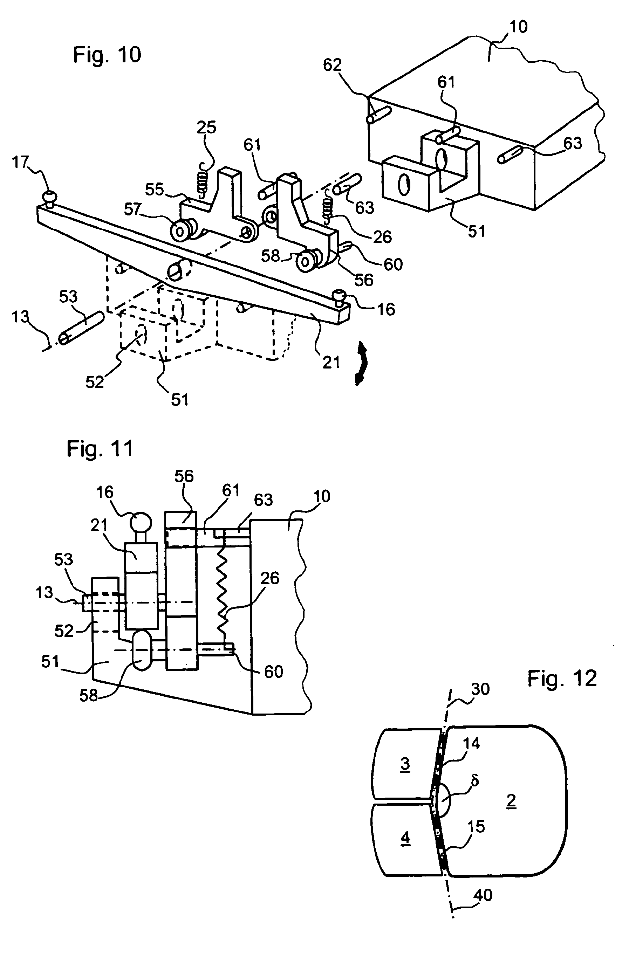

In Fig. 1 ist eine mehrteilige Sitzfläche 1 eines Zahnarztstuhls dargestellt, welche eine hintere Sitzplatte 2 zur Abstützung des Gesäßes sowie zur Abstützung der Beine mit der Sitzplatte 2 beweglich verbundene Beinauflagen 3, 4 aufweist, die um jeweils eine im Wesentlichen quer zur Sitzfläche 1 verlaufende Schwenkachse 30, 40 gegenüber der Sitzplatte 2 abgekippt werden können. Die beiden Schwenkachsen 30, 40 können nach diesem Ausführungsbeispiel gemäß Fig. 1 und 2 parallel angeordnet sein. Nach dem Ausführungsbeispiel gemäß Fig. 12 schließen die beiden Schwenkachsen 30, 40 einen Winkel δ von 162 Grad ein. Der Stuhl kann weiterhin eine Rückenlehne 7 zur Abstützung des Rückens aufweisen und ist mittels eines zentralen Stuhlbeins 8 mit einem Fußgestell 9 verbunden. Das Stuhlbein 8 ist dabei an einen gestrichelt dargestellten Hauptträger 10 unterhalb der Sitzplatte 2 befestigt, an dem auch eine Trägeranordnung 11 für die Beinauflagen 3, 4 befestigt ist, die nachfolgend näher erläutert wird. Dargestellt sind zwei Beinauflagen 3,4, die zueinander durch den Bereich 6 beabstandet sein können. Die Beinauflagen 3, 4 sind an der Vorderkante der Sitzplatte 2 angeordnet.

In der in Fig. 2 dargestellten Ansicht der Sitzfläche 1 von unten ist der Hauptträger 10 zu erkennen, auf dem die Sitzplatte 2 befestigt ist und der eine Befestigung 12 für das zentral angeordnete Stuhlbein 8 aufweist, das in Figur 1 dargestellt ist.

In dieser Ansicht erkennt man, dass die an der Vorderseite der Sitzplatte 2 angeordneten Beinauflagen 3, 4 über eine Trägeranordnung 11 an dem Hauptträger 10 gelagert sind, wobei die Trägeranordnung 11 um eine, unterhalb eines Bereiches 6 zwischen den Beinauflagen 3, 4 verlaufende Schwenkachse 13 herum schwenkbar ist, dargestellt durch den Pfeil 13.1.

Ein erstes Ausführungsbeispiel der Trägeranordnung 11 ist im Detail in Fig. 3 bis Fig. 6 dargestellt, zusammen mit dem Hauptträger 10. Ausgehend von diesem ist eine Wippe 21 als zentrales Teil der Trägeranordnung 11 über ein als Schraube 22 ausgebildetes Drehlager um die Schwenkachse 13 herum drehbar mit dem Hauptträger 10 verbunden, wobei im Hauptträger 10 Federkammern 23, 24 mit Federelementen 25, 26 vorgesehen sind, die mit in der Wippe 21 angeordneten, vorstehenden Bolzen 27, 28, die im zusammengebauten Zustand in die Federkammern 23, 24 hineinragen, zusammenwirken. Dies wird später näher beschrieben.

An den beiden Enden der Wippe 21 sind gegenüber der Längsachse 5 der Wippe 21 drehbar angeordnete Auflagearme 31, 32 vorgesehen, welche wiederum gegen eine Federkraft aus einer dargestellten Nulllage in Richtung des Pfeils 33 um die Längsachse 5 herum nach unten auslenkbar sind.

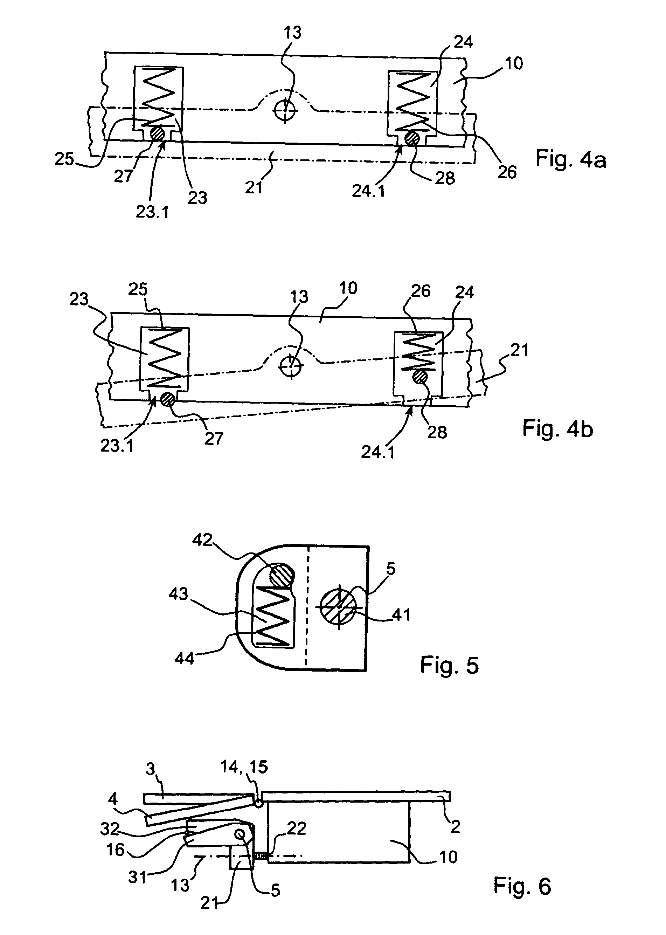

In Fig. 4A ist die Grundstellung der Wippe 21 in Bezug auf den Hauptträger 10 dargestellt, d.h., auf die Beinauflage 3, 4 wird keine Kraft nach unten ausgeübt. In dieser Grundstellung befindet sich die Wippe 21 in einer im Wesentlichen horizontalen Ausrichtung parallel zum Hauptträger und die der Wippe 21 zugeordneten Bolzen 27, 28 liegen in einer vertikalen Richtung gesehen gleichmäßig tief in den beiden Federkammern 23, 24 rechts und links der Schwenkachse 13 der Wippe 21 an den unter Vorspannung stehenden Federelementen 25, 26 an. Die beiden Federkammern 23, 24 weisen zum Einführen der Bolzen 27, 28 jeweils eine Federkammeröffnung 23.1, 24.1 auf.

In Fig. 4B ist die Wippe 21 in einer gegenüber dem Hauptträger 10 ausgelenkten Stellung dargestellt, wobei die linke Seite der Wippe 21 durch Belastung der vom Benutzer ausgesehen rechten Beinauflage 3 (Fig. 1) nach unten und dementsprechend die rechte Seite der Wippe 21 mit der Benutzer ausgesehenen linken Beinauflage 4 (Fig. 1) nach oben ausgelenkt ist. In der gezeigten Position ist der Bolzen 27 auf der linken Seite außerhalb der Federkammer 23 und der Bolzen 28 auf der rechten Seite wird gegen die Federkraft des Federelements 26 in das Innere der Federkammer 24 hineingedrückt. Ein mechanischer Anschlag der Wippe 21 in Richtung um die Schwenkachse 13 wird durch den je nach Schwenkrichtung der Wippe 21 eingreifenden Bolzen 27, 28 erreicht, der gegen das jeweilige Federelement 44, 45 und die jeweilige Federkammern 23, 24 drückt bzw. dort anschlägt.

Das hier dargestellte Federprinzip zur Federung und Rückstellung der Wippe von einer Grundstellung in eine Schrägstellung und zurück kann selbstverständlich im Rahmen des fachmännischen Könnens variiert werden, etwa durch Einsatz einer einzigen Drehfeder, die koaxial zu der Schwenkachse 13 angeordnet ist.

In Fig. 5 ist ein Schnitt entlang der Linie AA aus Fig. 3 dargestellt, um das zusätzliche im Wesentlichen vertikale Einfedern des Auflagearms 31 gegenüber der Wippe 21 zu verdeutlichen. Der Auflagearm 31 ist über einen Lagerzapfen 41 um die Achse 5 drehbar mit der Wippe 21 verbunden, wobei ein in dem Auflagearm 31 angeordneter, feststehender Bolzen 42 in eine Federkammer 43 am Ende der Wippe 21 eingreift. Der Bolzen 42 wirkt so auf ein Federelement 44 ein, dass ein Verschwenken des Auflagearms 31 um den Lagerzapfen 41 gegen die Federkraft des in der Federkammer 43 angeordneten Federelements 44 erfolgt. Ein mechanischer Anschlag des Auflagearms 31 in Richtung um die Achse 5 wird durch den Lagerzapfen 41 erreicht, der gegen das Federelement 44 und die Federkammer 43 drückt bzw. dort anschlägt. Entsprechend funktioniert der Anschlag des Auflagearms 32, jedoch sind die Federkammer und der Bolzen nicht dargestellt.

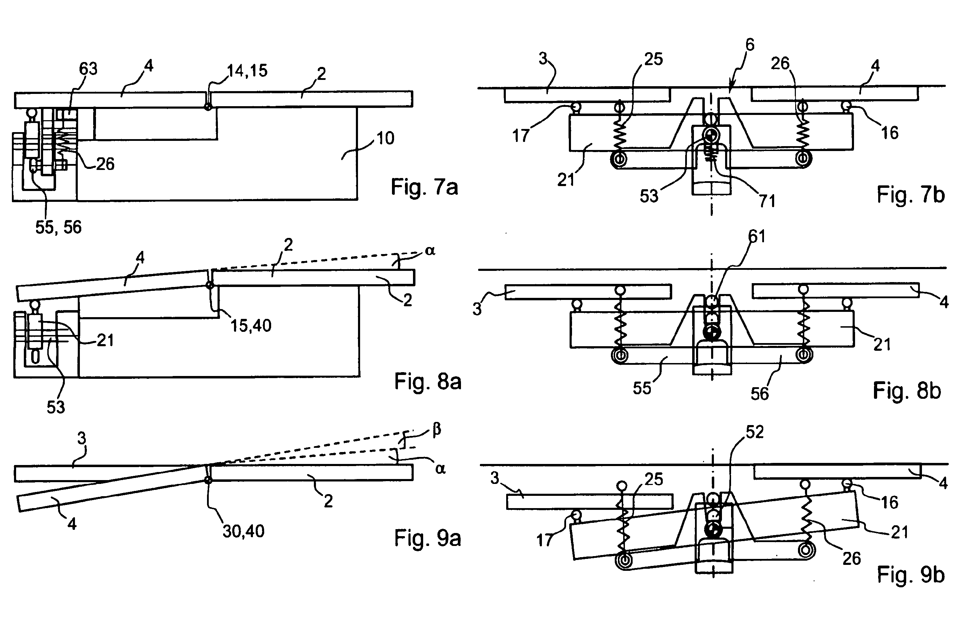

Die Federkraft des in dieser Federkammer 43 angeordneten Federelements 44 entspricht in etwa der Federkraft des an dem gegenüberliegenden Ende der Wippe 21 angeordneten, nicht dargestellten Federelements 45 des Auflagearms 32, wobei diese Federkraft deutlich kleiner ist als die Federkraft der Federelemente 25, 26 in den Federkammern 23, 24 des Hauptträgers 10. Dies bedeutet, dass eine Belastung der Beinauflage 3, 4(Fig. 1) und damit eine Belastung der Auflagearme 31, 32 zunächst ein Einfedern der beiden in den Federkammern 43 der Wippe 21 untergebrachten Federelemente 44 bewirkt, so dass beide Beinauflagen 3, 4 ausgehend von einer in etwa horizontalen Position (Fig. 7A, 7B) der Sitzplatte 2 jeweils unabhängig von einander um einen Winkel α (Fig. 8A, 8B) weit nach unten geneigt werden können. Danach schlagen die Auflagearme 31, 32, wie gemäß Fig. 5 beschrieben, an. Der Winkel α beträgt in allen Ausführungsbeispielen maximal 9 Grad, insbesondere zwischen 2 Grad und 5 Grad.

Erst nach Erreichen einer Kraft, die der Vorspannung der Federelemente 25, 26 in den am Hauptträger 10 angeordneten Federkammern 23, 24 entspricht, kann die Wippe 21 bei weiterer Belastung einer der beiden Beinauflagen 3, 4 gegen die Federkraft einer der beiden in der Federkammer des Hauptträgers 10 angeordneten Federelemente 25, 26 um die Schwenkachse 13 verschwenkt werden.

In der in Fig. 6 dargestellten Seitenansicht wird das Zusammenspiel der am Hauptträger 10 angeordneten Sitzplatte 2 mit der über das Drehlager 22 verbundenen Wippe 21 sowie die dazugehörige Abstützung der Beinauflagen 3, 4 durch die Auflagearme 31, 32 verdeutlicht. Die Beinauflage 3, 4 verschwenkt um das Scharnier 14, 15 um eine Schwenkachse 30, 40 (Fig. 2) und neigt sich gleichzeitig um die Längsachse 5 der Wippe 21. Die Beinauflage 3, 4 liegt über ein Lager 16, 17 auf dem Auflagearm 31, 32 auf. Das Lager 16, 17 ermöglicht eine Relativbewegung zwischen der Beinauflage 3, 4 und dem Auflagearm 31, 32 oder der Wippe 21.

In den Fig. 7A bis Fig. 11 ist ein weiteres Ausführungsbeispiel einer Trägeranordnung 11 dargestellt.

Die Fig. 7A bis 9A zeigen eine Seitenansicht und die Fig. 7B bis 9b die jeweilige Frontansicht. Gemäß Fig. 7B sind die Beinauflagen 3, 4 über ein Scharnier 14, 15 mit dem Hauptträger 10 oder mit der Sitzplatte 2 verbunden, dargestellt in einer Grundposition "0" in horizontal fluchtender Ausrichtung. Eine erste Belastung einer oder beider Beinauflagen 3, 4 führt zu einem gleichmäßigen Abwinkeln beider Beinauflagen 3, 4 aus der Grundposition "0" um einen Winkel α in eine in Fig. 8A und 8B dargestellte erste Zwischenposition "1", wobei hier vorzugsweise ein Winkel α von 3 Grad möglich ist. Das Abwinkeln der jeweiligen Beinauflage (3,4)erfolgt um die Schwenkachse 30, 40 des Scharniers 14, 15.

Erst bei einer weiteren Belastung einer der beiden Beinauflagen 3, 4, hier der Beinauflage 3, wird eine Wippe 21 betätigt, die das Abwinkeln der einen Beinauflage 3 um einen Winkel β und das gleichzeitige Anheben der anderen Beinauflage 4 um den gleichen Winkelbetrag β zur Folge hat. Das Abwinkeln der Beinauflage 3 um den Winkel β gegenüber der Sitzplatte 2 führt zu der Endposition "2". Die angehobene Beinauflage 4 wird in dieser Position "2" nicht über die Sitzplatte 2 hinaus hochgehoben, dargestellt in Fig. 9A und 9B.

Dieses Verschwenken der Wippe 21 führt also beim Absenken der einen Beinauflage 3 um einen an den Winkel α anschließenden Winkel β (Fig. 9A, 9B) zu einem gleichzeitigem Anheben der anderen Beinauflage 4. Die geometrischen Verhältnisse können dabei so ausgebildet sein, dass in keinem Fall ein Aufstellen der sich hebenden Beinauflage 4 über die Neigung der Sitzplatte 2 hinaus stattfindet, so dass ein Aufstellen der Beinauflage 4 gegenüber der Sitzplatte 2 vermieden wird. Die Beinauflage 4 befindet sich in ihrer Endposition "2" gemäß Fig. 9B in der gleichen Ebene wie in der Grundposition "0" gemäß Fig. 7B. Hierbei ist der Winkel α gleich dem Winkel β.

Wie in Fig. 10 im Detail gezeigt, wird in diesem Ausführungsbeispiel die Wippe 21 über Federelemente 25, 26 und jeweils einen Bolzen 62, 63 direkt an dem Hauptträger 10 aufgehängt. Die Vertikalverschiebung der Wippe 21 wird unter Last begrenzt, hierzu ist am Hauptträger 10 ein Vertikalanschlag 51 vorgesehen, auf dem die Wippe 21 nach einer Belastung den an den Enden der Wippe 21 gelagerten Beinauflagen 3, 4 aufliegt. In dieser Position führt die Belastung einer Beinauflage 3 und das damit verbundene Absenken der Beinauflage 3 zu einer Anhebung der anderen Beinauflage 4, wie in Fig. 9B gezeigt.

Es hat sich als vorteilhaft herausgestellt, wenn die Wippe 21 in einer Längsnut 52 in einer horizontalen Ebene geführt wird, um sicher zu stellen, dass die Wippe 21 im Anschlag 51 die vorgesehene Position auch tatsächlich einnimmt und hält. Dazu ist ein als Drehlager ausgebildeter Bolzen 53 vorgesehen, der in der Längsnut 52 geführt ist. Die Wippe 21 kann dabei um die durch den Bolzen 53 verlaufende Achse 13 gedreht werden, auch wenn die Anschlagsposition erreicht ist.

Weiterhin hat sich herausgestellt, dass die Federelemente 25, 26 vorteilhafterweise auf zwei von einander unabhängig bewegbaren Mitnehmern 55, 56 einwirken, die beide an der Schwenkachse 13 der Wippe 21 über den Bolzen 53 mit derselben verbunden sind und um diese Schwenkachse 13 schwenkbar sind. Dabei sind Auflager 57, 58 vorgesehen, die die Wippe von unten abstützen.

Die beiden federbelasteten Mitnehmer 55, 56 halten dabei die Wippe 21 in einem Gleichgewicht, wobei jeder Mitnehmer 55, 56 bei Auslenkung der Wippe 21 unabhängig an dem über jeweils einen Befestigungsbolzen 60 verbundenen Federelement 25, 26 zieht. Dies wird durch einen mit dem Anschlag 51 zusammenwirkenden Bolzen 61 am Hauptträger 10 bewirkt. Der Bolzen 61 bewirkt, dass die beiden Mitnehmer 55, 56, die durch die Federelemente 25, 26 nach oben gezogen werden, in einer Grundstellung gehalten werden.

Die ganze Einheit aus Wippe 21 und Mitnehmern 55, 56 wird mittels des Bolzens 53 am Hauptträger 10 gelagert. Dieses System verhindert, dass sich die beiden Zugfedern 25, 26 ohne diese Einheit bei Belastung in Ihrer Wirkung gegenseitig aufheben und garantiert eine waagrechte Stellung der Wippe 21.

Die Wippenfunktion kann in diesem Fall erst erreicht werden, wenn die Wippe 21 bzw. der Bolzen 53 auf dem Anschlag 51 aufsitzt und wenn eine einseitige Kraft auf die Wippe 21 ausgeübt wird.

Das ganze Prinzip der separaten Absenkung der Beinauflagen beruht darauf, eine möglichst große Absenkung auf nur einer Seite zu erreichen, ohne die andere im entsprechenden Verhältnis anzuheben. Gemäß der Erfindung wird die jeweils andere Seite, nämlich um das Maß der zuerst ausgeführten Absenkung reduziert, so dass das andere Bein nicht übermäßig angehoben wird.

In Fig. 11 ist das Ausführungsbeispiel aus Fig. 10 im Bereich des Anschlags 51 in einer Seitenansicht im Detail dargestellt.

In dem am Hauptträger 10 befestigten Anschlag 51 ist die Trägeranordnung 11 in einer Seitenansicht dargestellt. Zu erkennen ist die Wippe 21, die an dem Bolzen 53 um die Achse 13 drehbar in einer Längsnut 52 des Anschlags 51 geführt und gelagert ist. Die Wippe 21 ist auf ihrer Unterseite durch ein Auflager 58 in Form einer Rolle abgestützt, welches wiederum mit einem Mitnehmer 56 verbunden ist, der ebenfalls an dem Bolzen 53 gelagert ist. Der Mitnehmer 56 ist seinerseits über einen Bolzen 60 und ein Federelement 26 an dem Hauptträger 10 federnd gelagert, so dass bei einer Verschiebung des Bolzens 53 mit seiner Achse 13 in der Längsnut 52 nach unten gegen die Federkraft des Federelements 26 gearbeitet werden muss.

In der dargestellten Stellung liegt der Mitnehmer 56 an dem Bolzen 61, der mit dem Hauptträger 10 verbunden ist, an. Bei einer nur einseitigen Belastung der Wippe 21 sorgt diese Lange des Mitnehmers 56 am Bolzen 61 dafür, dass die gemeinsame Schwenkachse 13 der Wippe 21 und des Mitnehmers 56 in der Längsnut 52 bis zum Erreichen des durch das Ende der Längsnut 52 gebildeten Anschlags nach unten gedrückt wird. Durch die Absenkung des Drehzentrums der Wippe 21 wird dafür gesorgt, dass das nicht belastete Ende der Wippe 21 zu weit nach oben angehoben wird.

Es ist selbstverständlich möglich, ein zusätzliches Federelement, etwa eine in Fig. 7B dargestellte Druckfeder 71, vorzusehen, um eine Absenkung der Schwenkachse 13 des Bolzens 53 bei Krafteinwirkung auf eine Beinauflage 3, 4 zu begünstigen, wobei die Federkraft dieses Federelements 71 stets kleiner sein muss als die Federkraft der an den Mitnehmern 55, 56 angreifenden Federelemente 25, 26. Dadurch wird ein stufenweises Zusammenspiel der einzelnen Federelemente 71; 25, 26 ermöglicht, nämlich bei einer ersten Krafteinleitung eine Absenkung der Schwenkachse 13 gegen die Federkraft des Federelements 71 bis zum Erreichen des Anschlags in der Längsnut 52 für die Verstellung und bei einer weiteren einseitigen Belastung mit einer weitergehenden zweiten Krafteinleitung eine Auslösung der Funktion der Wippe 21 mit einem Absenken des belasteten Endes und Anheben des unbelasteten Endes der Wippe 21 gegen die Federkraft des belasteten Federelements 25 bis zu einem weiteren Anschlag für die Verstellung der Wippe 21. Der Anschlag der Wippe 21 ist durch den Vertikalanschag 51 gebildet.

### Bezugszeichenliste

- 1: Sitzfläche
- 2: Sitzplatte
- 3: Beinauflage
- 4: Beinauflage
- 5: Längsachse
- 6: Bereich
- 7: Rückenlehne
- 8: Stuhlbein
- 9: Fußgestell
- 10: Hauptträger
- 11: Trägeranordnung
- 12: Befestigung
- 13: Schwenkachse, Drehachse
- 13.1: Pfeil
- 14: Scharnier
- 15: Scharnier
- 16: Lager
- 17: Lager
- 21: Wippe
- 22: Drehlager, Schraube
- 23: Federkammer
- 23.1: Federkammeröffnung
- 24: Federkammer
- 24.1: Federkammeröffnung
- 25: Federelement, Zugfeder
- 26: Federelement, Zugfeder
- 27: Bolzen
- 28: Bolzen
- 30: Schwenkachse
- 31: Auflagearme
- 32: Auflagearme
- 33: Pfeil
- 40: Schwenkachse
- 41: Lagerzapfen
- 42: Bolzen
- 43: Federkammer
- 44: Federelement
- 45: Federelement
- 51: Vertikalanschlag
- 52: Längsnut
- 53: Drehlager, Bolzen
- 55: Mitnehmer
- 56: Mitnehmer
- 57: Auflager
- 58: Auflager
- 60: Befestigungsbolzen
- 61: Bolzen
- 62: Bolzen
- 63: Bolzen
- 71: Federelement
- α: Winkel
- β: Winkel
- δ: Winkel

## Patentansprüche

1. Stuhl, insbesondere ein Zahnarztstuhl, aufweisend eine im wesentlichen horizontal ausgerichtete Sitzfläche (1) mit einer Sitzplatte (2) zur Aufnahme des Gesäßes einer Person und weiterhin umfassend mindestens zwei sich an die Sitzplatte (2) anschließende Beinauflagen (3, 4), die gegenüber der Sitzplatte (2) um einen Winkel bewegbar sind, wobei sich beide Beinauflagen (3, 4) auf einer unterhalb derselben angeordneten und mit der Sitzplatte (2) zumindest mittelbar verbundenen Wippe (21) abstützen, wobei die Wippe (21) beim Verschwenken einer der beiden Beinauflagen (3) nach unten eine Anhebung der anderen Beinauflage (4) relativ zu der nach unten verschwenkten Beinauflage (3) nach oben bewirkt, **dadurch gekennzeichnet, dass** beide Beinauflagen (3, 4) gegenüber der Sitzplatte (2) um einen Winkel (α) gleichzeitig nach unten und eine der beiden Beinauflagen (3, 4) um einen an den Winkel (α) anschließenden Winkel (β) neigbar ist, wobei die Wippe (21) beim Verschwenken einer der beiden Beinauflagen (3) über den Winkel (α) hinaus nach unten eine Anhebung der anderen Beinauflage (4), relativ zu der über den Winkel (α) hinaus nach unten verschwenkten Beinauflage (3) nach oben bewirkt.

2. Stuhl nach Anspruch 1, **dadurch gekennzeichnet, dass** die Wippe (21) über ein Drehlager (22; 53) an einem Hauptträger (10) der Sitzplatte (2) um eine Schwenkachse (13) schwenkbar gelagert ist.

3. Stuhl nach einem der Ansprüche 1 oder 2, **dadurch gekennzeichnet, dass** die beiden Beinauflagen (3, 4) jeweils gegen eine Federkraft eines Federelements (44, 45) gegenüber der Sitzplatte (2) verschwenkbar sind und dass die Wippe (21) gegen eine Federkraft zumindest eines Federelements (25, 26) gegenüber der Sitzplatte (2)verschwenkbar ist, wobei die zur Verstellung der Beinauflagen (3, 4) erforderliche Kraft kleiner als die zur Verstellung der Wippe (21) erforderliche Kraft ist.

4. Stuhl nach Anspruch 3, **dadurch gekennzeichnet, dass** die Wippe (21) Auflagearme (31, 32) für mindestens jeweils eine Beinauflage (3, 4) aufweist, wobei die Auflagearme (31, 32) in ihrer Höhe gegenüber der Wippe (21) verstellbar sind.

5. Stuhl nach Anspruch 3 oder 4 und 2, **dadurch gekennzeichnet, dass** die Wippe (21) zwei Bolzen (27, 28) aufweist, über die die Wippe (21) an dem jeweiligen Federelement (25, 26) angelenkt ist, wobei das Federelement (25, 26) in einer Federkammer (23, 24) des Hauptträgers (10) angeordnet ist und der Bolzen (27, 28) über eine Federkammeröffnung (23.1, 24.1) in einer Richtung um die Schwenkachse (13) in die Federkammer (23, 24) einführbar ist.

6. Stuhl nach Anspruch 4 und 5, **dadurch gekennzeichnet, dass** der Auflagearm (31, 32) über einen Lagerzapfen (41) um eine Achse (5) schwenkbar an der Wippe (21) gelagert ist.

7. Stuhl nach einem der Ansprüche 1 oder 2, **dadurch gekennzeichnet, dass** die beiden Beinauflagen (3, 4) jeweils gegen eine Federkraft eines Federelements (25, 26) gegenüber der Sitzplatte (2) verschwenkbar sind und dass die Wippe (21) zumindest gegen eine Federkraft zumindest eines Federelements (71) gegenüber der Sitzplatte (2) höhenverstellbar ist, wobei die zur Verstellung der Beinauflagen (3, 4) erforderliche Kraft kleiner als die zur Verstellung der Wippe (21) erforderliche Kraft ist.

8. Stuhl nach Anspruch 7 und 2, **dadurch gekennzeichnet, dass** die Wippe (21) beidseitig der Schwenkachse (13) auf jeweils einem Mitnehmer (55, 56) auflegbar ist, wobei die Mitnehmer (55, 56) um die Schwenkachse (13) oder parallel zu der Schwenkachse (13)über einen als Drehlager ausgebildeten Bolzen (53) schwenkbar gelagert und in Richtung um seine Schwenkachse (13) über die Federelemente (25, 26) gegenüber der Sitzplatte (2) oder dem Hauptträger (10) vorspannbar sowie in Richtung der Schwenkachse (13) gegen einen am Hauptträger (10) befestigten Bolzen (61) anschlagbar sind.

9. Stuhl nach Anspruch 8, **dadurch gekennzeichnet, dass** die Wippe (21) höhenverstellbar ist und für die Höhenverstellung über das Drehlager in einer vertikal verlaufenden Längsnut (52) am Hauptträger (10) gelagert ist und die Wippe (21) oder das Drehlager über das Federelement (71) in Richtung der Längsnut (52) vorspannbar ist, wodurch ein gleichzeitiges Absenken der beiden Beinauflagen (3, 4) ermöglicht wird.

10. Stuhl nach einem der Ansprüche 1 bis 9, **dadurch gekennzeichnet, dass** die Verstellung der Beinauflagen (3, 4) durch einen oder mehrere Anschläge begrenzt ist und dass die Verstellung der Wippe (21) durch einen oder mehrere Anschläge begrenzt ist, wobei der oder die Anschläge zur Verstellung der Beinauflagen (3, 4) von dem oder den Anschlägen zur Verstellung der Wippe (21) verschieden sind.

11. Stuhl nach einem der Ansprüche 1 bis 10, **dadurch gekennzeichnet, dass** die jeweilige Beinauflage (3, 4) über ein Scharnier (14, 15) jeweils um eine Schwenkachse (30, 40) an der Sitzplatte (2) schwenkbar gelagert ist und dass die beiden Schwenkachsen (30, 40) koaxial oder in einem Winkel (δ) zwischen 135 und 179 Grad, zueinander angeordnet sind.

12. Stuhl nach Anspruch 1 und 3 oder 7, **dadurch gekennzeichnet, dass** das Maß des Winkels (α) und des Winkels (β) und/oder die Federkräfte manuell einstellbar sind.

13. Stuhl nach einem der Ansprüche 1 bis 12, weiter aufweisend ein Stuhlbein (8) sowie ein Fußgestell (9) und eine Rückenlehne (7).

14. Verfahren zum Verstellen der Neigungswinkel von den beiden Beinauflagen (3, 4) eines Stuhls nach einem der vorstehenden Ansprüche, **gekennzeichnet durch** folgende Verfahrensschritte und ausgehend von einer Grundposition "0", in der beide Beinauflagen (3, 4) den geringsten Neigungswinkel gegenüber der Sitzplatte (2) aufweisen:
a) **durch** die Belastung einer oder beider Beinauflagen (3, 4) werden beide Beinauflagen (3, 4) in Bezug zu der Sitzplatte (2) entgegen einer Federkraft gleichzeitig um einen Winkel (α) von maximal 9 Grad in eine Zwischenposition "1" geneigt;
b) **durch** eine weitere Belastung einer der beiden Beinauflagen (3) und einer daraus resultierenden Bewegung der Beinauflage (3) entgegen einer Federkraft über den Winkel (α) hinaus nach unten, wird die andere Beinauflage (4), relativ zu der über den Winkel (α) hinaus nach unten geneigten Beinauflage (3) nach oben bewegt;
c) die eine Beinauflage (3) wird so lange nach unten bewegt, bis die andere nach oben bewegte Beinauflage (4) den gleichen Neigungswinkel gegenüber der Sitzplatte (2) aufweist wie in der Grundposition "0", bis eine Endposition "2" erreicht ist;
d) wenn die Beinauflagen (3, 4) die Endposition "2" erreicht haben, wird **durch** mehrere Anschläge vermieden, dass sich die eine Beinauflage (4) weiter anhebt und dass sich die andere Beinauflage (3) weiter absenkt.

15. Verfahren nach Anspruch 14, **dadurch gekennzeichnet, dass** das Maß des Winkels (α) und des Winkels (β) und/oder die Federkräfte manuell einstellbar sind.

## Claims

1. A chair, more particularly a dentist's chair, exhibiting a substantially horizontal sitting surface (1) comprising a seat board (2) for the accommodation of a person's buttocks, and further comprising at least two leg rests (3, 4) connected to said seat board (2) and displaceable relatively to said seat board (2) through an angle, both of said leg rests (3, 4) being supported on a rocker (21) disposed beneath them and at least indirectly connected to said seat board (2), which rocker (21), when one of the two leg rests (3) is pivoted downwardly, causes the other leg rest (4) to be raised relatively to the downwardly pivoted leg rest (3), **characterized in that** both leg rests (3, 4) can be tilted simultaneously downwardly relatively to said seat board (2) through an angle (□) and one of said two leg rests (3, 4) can be tilted through an angle (□) directly adjacent said angle (□) wherein said rocker (21), when one of said two leg rests (3) pivots downwardly beyond the range of said angle (□), causes said other leg rest (4) to rise relatively to said leg rest (3) pivoted downwardly beyond said angle (□)

2. The chair as defined in claim 1, **characterized in that** said rocker (21) is mounted via a pivot bearing (22; 53) on a main support (10) of said seat board (2) for rotation about a pivot axis (13).

3. The chair as defined in claim 1 or claim 2, **characterized in that** each of said two leg rests (3, 4) is pivotable against the spring force of a resilient element (44, 45) relatively to said seat board (2) and said rocker (21) is pivotable against the spring force of at least one resilient element (25, 26) relatively to said seat board (2), the force required to adjust said leg rests (3, 4) being less than the force required to adjust said rocker (21).

4. The chair as defined in claim 3, **characterized in that** said rocker (21) has supporting arms (31, 32) for at least one leg rest (3, 4), said supporting arms (31, 32) being vertically adjustable in relation to said rocker (21).

5. The chair as defined in claim 3 or in claims 4 and 2, **characterized in that** said rocker (21) has two bolts (27, 28), by means of which said rocker (21) is pivotally attached to said respective resilient element (25, 26), and said resilient element (25, 26) is disposed in a spring-holding chamber (23, 24) in said main support (10) and said bolt (27, 28) can be inserted into the spring-holding chamber (23, 24) in a direction around said swivel axis (13) via a spring-holding chamber opening (23.1, 24.1).

6. The chair as defined in claim 4 and claim 5, **characterized in that** said supporting arm (31, 32) is mounted on said rocker (21) via a journal (41) for rotation about an axis (5).

7. The chair as defined in claim 1 or claim 2, **characterized in that** each of said two leg rests (3, 4) is pivotable against an elastic force of a resilient element (25, 26) relatively to said seat board (2) and that said rocker (21) is vertically adjustable at least against an elastic force of at least one resilient element (71) relatively to said seat board (2), the force required to adjust said leg rests (3, 4) being less than the force required to adjust said rocker (21).

8. The chair as defined in claim 7 and claim 2, **characterized in that** said rocker (21) can be positioned on an entrainer (55, 56) on each side of said pivot axis (13), which entrainers (55, 56) are mounted for rotation about said pivot axis (13) or parallel to said pivot axis (13) via a bolt (53) forming a pivot bearing and can be biased relatively to said seat board (2) or said main support (10) in the direction around its pivot axis (13) via said resilient elements (25, 26) and can be stopped in the direction of said pivot axis (13) by a bolt (61) fixed to said main support (10).

9. The chair as defined in claim 8, **characterized in that** said rocker (21) is vertically adjustable and, for the purpose of vertical adjustment via said pivot bearing, is mounted in a vertically extending longitudinal groove (52) on said main support (10) and said rocker (21), or said pivot bearing can be biased by said resilient element (71) in the direction of said longitudinal groove (52), by which means simultaneous lowering of said two leg rests (3, 4) is made possible.

10. The chair as defined in any one of claims 1 to 9, **characterized in that** adjustment of said leg rests (3, 4) is restricted by one or more stops and adjustment of said rocker (21) is restricted by one or more stops, the stop(s) intended for adjustment of said leg rests (3, 4) being different from the stops intended for adjustment of said rocker (21).

11. The chair as defined in any one of claims 1 to 10, **characterized in that** the respective leg rests (3, 4) are each mounted for rotation via a hinge (14, 15) about a swivel axis (30, 40) on said seat board (2), and the two swivel axes (30, 40) are disposed coaxially or at an angle (□) of from 135 to 179 degrees relative to each other.

12. The chair as defined in claim 1 and 3 or 7, **characterized in that** the extent of the angle (□) and that of the angle (□) and/or the strength of the spring forces can be adjusted manually.

13. The chair as defined in any one of claims 1 to 12, further comprising a chair leg (8), a base plate (9), and a backrest (7).

14. A method for adjusting the angle of inclination of said two leg rests (3, 4) of a chair as defined in any one of the previous claims, **characterized by** the following method steps starting from a basic position "0", in which the two leg rests (3, 4) are at the smallest angle of inclination in relation to said seat board (2):
a) a load on one or both leg rests (3, 4) causes inclination of both leg rests (3, 4) in relation to said seat board (2) with counteraction of an elastic force and simultaneous tilting through an angle (□) of not more than 9 degrees to take up an intermediate position "1";
b) a further load on one of the two leg rests (3) and a concomitant downward movement of the leg rest (3) counteracting an elastic force to beyond the angle (□), causes the other leg rest (4) to move upwardly relatively to the leg rest (3) tilted downwardly to beyond the angle (□) ;
c) the leg rest (3) is lowered until the other, upwardly moving leg rest (4) assumes the same angle of inclination in relation to said seat board (2) as in the basic position "0", until an end position "2" is assumed;
d) when the leg rests (3, 4) have reached the end position "2", a plurality of stops prevents the one leg rest (4) from rising further and the other leg rest (3) from lowering further.

15. The method as defined in claim 14, **characterized in that** the extent of the angle (□) and that of the angle (□) and/or the strength of the spring forces can be adjusted manually.

## Revendications

1. Chaise, notamment chaise pour dentiste, présentant une surface d'assise (1) orientée sensiblement à l'horizontale avec un plateau d'assise (2) destiné à recevoir le postérieur d'une personne et comprenant en outre au moins deux supports de jambes (3, 4) se raccordant au plateau d'assise (2) et qui peuvent bouger par rapport au plateau d'assise (2) à raison d'un angle, les deux supports de jambes (3, 4) s'appuyant sur un basculeur (21) disposé en dessous de celui-ci et raccordé du moins indirectement au plateau d'assise (2), le basculeur (21), lors du pivotement vers le bas d'un des deux supports de jambes (3), provoquant un relèvement de l'autre support de jambe (4) vers le haut par rapport au support de jambe pivoté vers le bas (3), **caractérisée en ce que** les deux supports de jambes (3, 4) sont inclinables par rapport au plateau d'assise (2) à raison d'un angle (α) et que, simultanément, un des deux supports de jambes (3, 4) est inclinable vers le bas à raison d'un angle (β) se raccordant à l'angle (α), le basculeur (21), lors du pivotement vers le bas d'un des deux supports de jambes (3) au-delà de l'angle (α), provoquant un relèvement de l'autre support de jambe (4) vers le haut par rapport au support de jambe pivoté vers le bas (3) au-delà de l'angle (α).

2. Chaise selon la revendication 1, **caractérisée en ce que** le basculeur (21) s'appuie de manière pivotante via un palier rotatif (22 ; 53) sur un support principal du plateau d'assise (2) autour d'un axe de pivotement (13).

3. Chaise selon une des revendications 1 ou 2, **caractérisée en ce que** les deux supports de jambes (3, 4) peuvent pivoter respectivement à l'encontre d'une force de ressort d'un élément à ressort (44, 45) par rapport au plateau d'assise (2) et que le basculeur (21) peut pivoter à l'encontre d'une force de ressort d'au moins un élément à ressort (25, 26) par rapport au plateau d'assise (2), la force nécessaire au réglage des supports de jambes (3, 4) étant inférieure à celle nécessaire au réglage du basculeur (21).

4. Chaise selon la revendication 3, **caractérisée en ce que** le basculeur (21) présente des bras d'appui (31, 32) pour au moins respectivement un support de jambe (3, 4), les bras d'appui (31, 32) étant réglables en hauteur par rapport au basculeur (21).

5. Chaise selon les revendications 3 ou 4 et 2, **caractérisée en ce que** le basculeur (1) présente deux goujons (27, 28) par lesquels le basculeur (1) est articulé à l'élément à ressort respectif (25, 26), l'élément à ressort (25, 26) étant disposé dans une chambre à ressort (23, 24) du support principal (10) et le goujon (27, 28) pouvant être introduit par une ouverture de la chambre à ressort (23.1, 24.1) dans un sens autour de l'axe de pivotement (13) dans la chambre à ressort (23, 24).

6. Chaise selon les revendications 4 et 5, **caractérisée en ce que** le bras d'appui (31, 32) s'appuie de manière pivotante sur le basculeur (21) par un tourillon d'appui (41) autour d'un axe (5).

7. Chaise selon une des revendications 1 ou 2, **caractérisée en ce que** les deux supports de jambes (3, 4) peuvent pivoter respectivement à l'encontre d'une force de ressort d'un élément à ressort (25, 26) par rapport au plateau d'assise (2) et que le basculeur (21) est réglable en hauteur du moins à l'encontre d'une force de ressort d'au moins un élément à ressort (71) par rapport au plateau d'assise (2), la force nécessaire au réglage des supports de jambes (3, 4) étant inférieure celle nécessaire au réglage du basculeur (21).

8. Chaise selon les revendications 7 et 2, **caractérisée en ce que** le basculeur (21) peut être posé des deux côtés de l'axe de pivotement (13) respectivement sur un entraîneur (55, 56), les entraîneurs (55, 56) s'appuyant de manière pivotante autour de l'axe de pivotement (13) ou parallèlement à l'axe de pivotement (13) par un goujon (53) conformé en tant que palier pivotant et pouvant être précontraints dans le sens entourant son axe de pivotement (13) via les éléments à ressort (25, 26) par rapport au plateau d'assise (2) ou au support principal (10) et pouvant buter en direction de l'axe de pivotement (13) contre un goujon (61) fixé au support principal (10).

9. Chaise selon la revendication 8, **caractérisée en ce que** le basculeur (21) est réglable en hauteur et, pour son réglage en hauteur, s'appuie via le palier pivotant dans une rainure longitudinale (52) s'étendant verticalement sur le support principal (10) et que le basculeur (21) ou le palier pivotant peut être précontraint via l'élément à ressort (71) en direction de la rainure longitudinale (52), ce qui permet un abaissement simultané des deux supports de jambes (3, 4).

10. Chaise selon une des revendications 1 à 9, **caractérisée en ce que** le réglage des supports de jambes (3, 4) est limité par une ou plusieurs butées et que le réglage du basculeur (21) est limité par une ou plusieurs butées, la ou les butées permettant le réglage des supports de jambes (3, 4) étant différentes de la ou des butées permettant le réglage du basculeur (21).

11. Chaise selon une des revendications 1 à 10, **caractérisée en ce que** le support de jambe respectif (3, 4) s'appuie de manière pivotante via une charnière (14, 15) respectivement autour d'un axe de pivotement (30, 40) au niveau du plateau d'assise (2) et que les deux axes de pivotement (30, 40) sont disposés coaxialement ou suivant un angle (δ) compris entre 135 et 179 degrés l'un par rapport à l'autre.

12. Chaise selon les revendications 1 et 3 ou 7, **caractérisée en ce que** la dimension de l'angle (α) et de l'angle (δ) et/ou les forces de ressort sont réglables manuellement.

13. Chaise selon les revendications 1 à 12, présentant en outre un pied de chaise (8) et un piètement (9) et un dossier (7).

14. Procédé de réglage de l'angle d'inclinaison des deux supports de jambes (3, 4) d'une chaise selon une des revendications précédentes, **caractérisé par** les étapes opératoires suivantes et partant d'une position de base « 0 » dans laquelle les deux supports de jambes (3, 4) présentent le plus faible angle d'inclinaison par rapport au plateau d'assise (2) :
a) du fait de la charge d'un ou des deux supports de jambes (3, 4), les deux supports de jambes (3, 4) s'inclinent simultanément à l'encontre d'une force de ressort par rapport au plateau d'assise (2) à raison d'un angle (α) de 9 degrés au maximum pour prendre une position intermédiaire « 1 » ;
b) du fait d'une autre charge d'un des deux supports de jambes (3) et d'un mouvement vers le bas en résultant du support de jambe (3) à l'encontre d'une force de ressort au-delà de l'angle (α), l'autre support de jambe (4) se déplace vers le haut par rapport au support de jambe (3) incliné vers le bas au delà de l'angle (α) ;
c) un support de jambe (3) se déplace vers le bas jusqu'à ce que l'autre support de jambe (4) se déplaçant vers le haut présente le même angle d'inclinaison par rapport au plateau d'assise (2) que dans la position « 0 » jusqu'à ce qu'une position « 2 » soit atteinte ;
d) lorsque les supports de jambe (3, 4) ont atteint la position terminale « 2 », plusieurs butées évitent qu'un support de jambe (4) continue à se lever et que l'autre support de jambe (3) continue à s'abaisser.

15. Procédé selon la revendication 14, **caractérisé en ce que** la dimension de l'angle (α) et de l'angle (β) et/ou les forces de ressort sont réglables manuellement.
